# EUROPEAN PATENT APPLICATION

(11) **EP 3 534 369 A1**
(43) Date of publication of application: **04.09.2019**
(21) Application number: 19159568.5
(22) Date of filing: 27.02.2019
(51) Int. Cl.: G16H 10/60, G16H 40/20, G16H 10/40, G16H 20/00

(54) **PROACTIVE FOLLOW-UP OF CLINICAL FINDINGS**

(30) Priority: 01.03.2018 US 201815909336
(71) Applicant: Siemens Medical Solutions USA, Inc., Malvern, PA 19355 (US)
(72) Inventor: Frandsen, Tim, Germantown, TN 38139 (US); Iwase, Akio, Brisbane, CA 94005 (US); Furci, Nick, South San Francisco, CA 94080 (US)
(74) Representative: Patentanwälte Bals & Vogel

(57) **Abstract**

A framework for facilitating proactive follow-up of clinical findings. In accordance with one aspect, one or more clinical findings that require follow-up are extracted from one or more documents. One or more follow-up activities that are relevant to the one or more clinical findings are determined. Priority levels may be generated for the one or more follow-up activities. The one or more clinical findings, follow-up activities and priority levels may then be displayed in a user interface. A notification may be sent to a recipient to initiate at least one of the one or more follow-up activities.

## Description

### TECHNICAL FIELD

The present disclosure generally relates to digital data processing, and more particularly to a framework for facilitating proactive follow-up of clinical findings.

### BACKGROUND

Failure to follow-up on abnormal test results is a critical problem in healthcare, whether it be incidental findings identified in radiology exams, test results associated with the management of chronic disease, or follow-up on abnormal screening results. To demonstrate the scope of the problem, a systematic review of numerous studies has been conducted. See Callen JL, Westbrook JI, Georgiou A, et al., Failure to follow-up test results for ambulatory patients: a systematic review, J. Gen. Intern. Med. 2012;27:1334-48, which is herein incorporated by reference. The review reported quantitative evidence of the number of tests not followed up for patients attending ambulatory settings, which demonstrated that up to 35.7% of radiology studies did not have appropriate follow-up by the ordering provider. This is a problem that needs to be addressed, but current processes are varied and generally unsystematic. It is especially problematic with incidental findings, as demonstrated by a systematic review of various articles, which reported a frequency of incidental findings in imaging examinations being as high as 31%, with 35% not receiving clinical follow-up. See Lumbreras B, Donat L, Hernandez-Aguado I., Incidental findings in imaging diagnostic tests: a systematic review, Br. J. Radiol., 2010;83(988):276-89, which is herein incorporated by reference.

Another review of computed tomographic (CT) pulmonary angiographies ordered in the emergency department noted that in 9.9% of studies, pulmonary nodules were found. See Blagev DP, Lloyd JF, Conner K, et al., Follow-up on incidental pulmonary nodules and the radiology report, J. Am. Coll. Radiol., 2014;11:378-83, which is herein incorporated by reference. However, follow up for those nodules was extremely poor, with less than a third of those patients having appropriate lung nodule follow-up imaging according to the Fleischner Society guidelines.

In national surveys, 39-73% of radiologists had non-concordance with guidelines with regards to follow-up recommendations. See Eisenberg RL, Bankier AA, Boiselle PM, Compliance with Fleischner Society guidelines for management of small lung nodules: a survey of 834 radiologists, Radiology 2010;255:218-24; and Esmaili A, Munden RF, Mohammed TL, Small pulmonary nodule management: a survey of the members of the Society of Thoracic Radiology with comparison to the Fleischner Society guidelines, J. Thorac. Imaging 2011;26:27-31, which are herein incorporated by reference. This highlights the importance of a system's ability to communicate recommendations that are consistent with the relevant guidelines.

### SUMMARY

Described herein is a framework for facilitating proactive follow-up of clinical findings. In accordance with one aspect, one or more clinical findings that require follow-up are extracted from one or more documents from electronic medical records. One or more follow-up activities that are relevant to the one or more clinical findings are determined. Priority levels may be generated for the one or more follow-up activities. The one or more clinical findings, follow-up activities and priority levels may then be presented in a user interface. A notification may be sent to a recipient to initiate at least one of the one or more follow-up activities.

According to a first aspect of the invention a clinical follow-up system is provided, the clinical follow-up system comprising: a non-transitory memory device for storing computer readable program code; and a processor device in communication with the memory device, the processor being operative with the computer readable program code to perform steps including: extracting one or more clinical findings that require follow-up from one or more documents, determining one or more follow-up activities that are relevant to the one or more clinical findings, generating one or more priority levels for the one or more follow-up activities, generating a user interface that presents the one or more clinical findings, the one or more follow-up activities and the one or more priority levels, and sending a notification to a recipient to initiate at least one of the one or more follow-up activities.

Preferred is a system, wherein the processor is operative with the computer readable program code to extract the one or more clinical findings that require follow-up from the one or more documents using structured data capture, Natural Language Processing (NLP) techniques, or a combination thereof.

Further, a system is preferred, wherein the processor is operative with the computer readable program code to further validate the one or more extracted clinical findings by analyzing patient factors in the one or more documents that provides relevant clinical context.

According to a second aspect of the invention a computer-implemented method is provided, the computer-implemented method comprising: extracting one or more clinical findings that require follow-up from one or more documents; determining one or more follow-up activities that are relevant to the one or more clinical findings; sending a first notification to a recipient to initiate at least one of the one or more follow-up activities; monitoring a medical record database to determine whether the at leavst one of the one or more follow-up activities has occurred; and sending a second notification in response to occurrence of the at least one of the one or more follow-up activities.

Preferred is a method, wherein extracting the one or more clinical findings that require follow-up from the one or more documents comprises extracting the one or more clinical findings that require follow-up from one or more unstructured medical records, and/or Further, a system is preferred, wherein extracting the one or more clinical findings that require follow-up from the one or more documents comprises using structured data capture, Natural Language Processing (NLP) techniques or a combination thereof, and/or wherein extracting the one or more clinical findings that require follow-up from the one or more documents comprises extracting an incidental finding, abnormal results from diagnostic tests, a medical condition that requires routine clinical follow-up activities for management, or a combination thereof.

Preferred is a method, wherein determining the one or more follow-up activities that are relevant to the one or more clinical findings comprises extracting a recommendation for the one or more follow-up activities from the one or more documents, and/or wherein determining the one or more follow-up activities that are relevant to the one or more clinical findings comprises generating a recommendation for the one or more follow-up activities in accordance with relevant guidelines for the one or more clinical findings. Preferred is further a method, the method further comprises generating one or more priority levels for the one or more follow-up activities, in particular wherein generating one or more priority levels for the one or more follow-up activities comprises generating the one or more priority levels based on likelihood of the one or more follow-up activities not occurring.

Preferred is a method, the method further comprises presenting the one or more clinical findings and the one or more follow-up activities in a graphical representation displayed at a user interface, in particular, wherein the method further comprises enabling a user to review, confirm, remove or modify the one or more clinical findings or follow-up activities via the user interface.

Preferred is a method, wherein the method further comprises enabling a user to assign ownership of the one or more follow-up activities via the user interface.

Preferred is further a method, wherein the graphical representation comprises a worklist that identifies follow-up activities for only patients under a user's care.

Further, a method is preferred, wherein the method further comprises sorting the follow-up activities identified by the worklist according to priority levels.

Preferred is a method, wherein the method further comprises automatically assigning ownership of the one or more follow-up activities to one or more responsible care providers.

Preferred is a method, wherein sending the first notification to the recipient to initiate the at least one of the one or more follow-up activities comprises sending the first notification that enables the recipient to initiate automatic generation of an order for the at least one of the one or more follow-up activities.

Further, a method is preferred, wherein monitoring the medical record database to determine whether the at least one of the one or more follow-up activities has occurred comprises monitoring the medical record database to determine whether an order for the follow-up activity has been placed.

According to a third aspect of the invention one or more non-transitory computer readable media is provided, embodying a program of instructions executable by machine to perform steps comprising: extracting one or more clinical findings that require follow-up from one or more documents; determining one or more follow-up activities that are relevant to the one or more clinical findings; sending a first notification to a recipient to initiate at least one of the one or more follow-up activities; monitoring a medical record database to determine whether the at least one of the one or more follow-up activities has occurred; and sending a second notification in response to occurrence of the at least one of the one or more follow-up activities.

In the following description, numerous specific details are set forth such as examples of specific components, devices, methods, etc., in order to provide a thorough understanding of implementations of the present framework. It will be apparent, however, to one skilled in the art that these specific details need not be employed to practice implementations of the present framework. In other instances, well-known materials or methods have not been described in detail in order to avoid unnecessarily obscuring implementations of the present framework. While the present framework is susceptible to various modifications and alternative forms, specific embodiments thereof are shown by way of example in the drawings and will herein be described in detail. It should be understood, however, that there is no intent to limit the invention to the particular forms disclosed, but on the contrary, the intention is to cover all modifications, equivalents, and alternatives falling within the spirit and scope of the invention. Furthermore, for ease of understanding, certain method steps are delineated as separate steps; however, these separately delineated steps should not be construed as necessarily order dependent in their performance.

Unless stated otherwise as apparent from the following discussion, it will be appreciated that terms such as "segmenting," "generating," "registering," "determining," "aligning," "positioning," "processing," "computing," "selecting," "estimating," "detecting," "tracking" or the like may refer to the actions and processes of a computer system, or similar electronic computing device, that manipulates and transforms data represented as physical (e.g., electronic) quantities within the computer system's registers and memories into other data similarly represented as physical quantities within the computer system memories or registers or other such information storage, transmission or display devices. Embodiments of the methods described herein may be implemented using computer software. If written in a programming language conforming to a recognized standard, sequences of instructions designed to implement the methods can be compiled for execution on a variety of hardware platforms and for interface to a variety of operating systems. In addition, implementations of the present framework are not described with reference to any particular programming language. It will be appreciated that a variety of programming languages may be used.

While systems to identify and manage critical test results are well-established, there is currently no automated system infrastructure to track and identify non-critical test results and unexpected findings requiring follow-up in 6-12 months, as these follow-up events frequently fail to occur. Although there may be several reasons for a lack of follow-up, one explanation may be that the findings and suggested follow-up activities are typically found in unstructured clinical reports. Unstructured data does not have a schema or other form of data model, making it difficult to identify actionable information related to follow-up recommendations. However, such data provides tremendous value in enabling effective follow-up. Therefore, the ability to unlock this data to electronically identify findings and recommendations in unstructured data can reduce the chance that recommended follow-up activities in these reports are not overlooked by physicians.

The current standard of care for communicating diagnostic imaging results is provided by the American College of Radiology (ACR) Practice Parameter for Communication of Diagnostic Imaging Findings 2014. According to this standard of care, the interpreting physician should expedite delivery of a diagnostic report in a manner that reasonably ensures timely receipt of the findings, where the communication is usually sent to the ordering physician or his/her designee. This approach is focused on the delivery of the diagnostic report, rather than a workflow aimed at ensuring follow-up events occur. Moreover, this approach is inherently flawed in that diagnostic reports are communicated to care providers who are often not directly involved in the longer-term care of the patient, or may be specialized in a clinical area that is unrelated to the finding requiring follow-up, while those providers who are directly involved (and are therefore most likely to order follow-up examinations) are not included in this workflow. As a result, there must be a "hand off" between providers, which can be inconsistent or even non-existent. Additionally, focusing on the communication of the radiology report is not effective due to the nature of the radiology report itself. The majority of reports are not structured, thereby requiring the receiving physician to take time to identify the findings of interest and recommended follow-up, which are often buried as free text in the body of the reports. This is particularly problematic in the case of findings that are unrelated to the purpose for the examination.

The Electronic Medical Record (EMR) may be used to alert providers that a follow-up activity is recommended, but may not be effective due to "alert fatigue" (i.e., when healthcare providers receive more alerts for their patients than they are able to reasonably act upon). A significant number of physicians have reported having personally missed results that led to delayed care that resulted from alert fatigue. In addition, it is up to each physician's discretion in how and whether to utilize the EMR for activity reminders. This variability in workflow often leads to the inability to track and manage follow-ups at a population level, and in some cases results in follow-up actions being overlooked when trying to coordinate care with other clinicians. Moreover, many health systems have multiple disparate EMR solutions which limit the ability for the EMR to be a viable solution to improve patient follow-up compliance.

Other most commonly utilized solutions track follow-up events manually (e.g., using Microsoft's Excel spreadsheets). There are also relatively primitive solutions that have been developed internally by the institution, either independently or in collaboration with the EMR vendor, which lack automated workflow capabilities and rules engines required to efficiently manage patients.

A framework for facilitating proactive follow-up of clinical findings is presented herein. In accordance with one aspect, the framework automatically identifies clinical findings that require follow-up activities, even those that are buried in the text of unstructured reports. This helps ensure that clinical findings are not missed as a result of being difficult to identify in the document text. Current solutions focus on communicating diagnostic reports as-is, rather than highlighting the most relevant details. The present framework eliminates a potential source of medical errors that is currently not addressed.

Another aspect of the framework automatically extracts recommendations for follow-up activities from documents (e.g., diagnostic reports), and if such recommendations do not exist, the framework may generate recommendations based on preferences configured by the user. Current solutions typically do not provide follow-up recommendations unless specifically sought by the responsible provider. The workflow facilitated by the present framework advantageously eliminates hand-offs between care providers, thereby eliminating another potential source of error. Each follow-up activity may be assigned to the most relevant clinical care provider. Follow-up activities may be prioritized, thereby increasing the efficiency of care coordination. Prioritization criteria may be configured. Examples include, but are not limited to, the use of predictive modeling to prioritize follow-up activities based on risk or likelihood that a patient will fail to follow-up.

Yet another aspect of the present framework generates a user interface that automates the workflow of the clinical user. The user interface enables the clinical user to configure various rules, such as the care provider who is assigned as being responsible for the follow-up (i.e., owner of follow-up activity), who gets notified that a follow-up activity has been identified, if orders are placed in the EMR for the recommended follow-up activity, view notifications (or reminders) when follow-up activities are past due, etc. In some implementations, the user interface enables a user to modify one or more of the follow-up activities to suit their preferences. The user may also communicate information to other users via, for example, activity notes related to that patient. The user may further indicate completion of the follow-up activity via the user interface. Alternatively, the framework may automatically detect the completion of the follow-up activity through, for example, data feeds from electronic systems utilized for ordering, scheduling, performing medical examinations or a combination thereof.

The user interface serves to improve the efficiency of managing patients and reduce medical errors that are the result of inconsistent communication. Additionally, the framework may proactively monitor the medical record database (e.g., EMR) to determine if follow-up activities or events have occurred, send notifications and/or initiate proactive activities if these follow-up activities do not occur in accordance with the recommendations. These and other exemplary advantages and features will be described in more detail in the following description.

### BRIEF DESCRIPTION OF THE DRAWINGS

A more complete appreciation of the present disclosure and many of the attendant aspects thereof will be readily obtained as the same becomes better understood by reference to the following detailed description when considered in connection with the accompanying drawings.
FIG. 1 shows a block diagram illustrating an exemplary system;
FIG. 2 shows an exemplary method performed by a computer system;
FIG. 3 shows an exemplary decision tree for clinical knowledge extraction;
FIG. 4 shows an exemplary workflow for a user who is the rendering care provider;
FIG. 5 shows an exemplary method of automatically assigning ownership of a follow-up activity to a responsible care provider; and
FIG. 6 shows an exemplary workflow for a user who is the owner of a follow-up activity.

### DETAILED DESCRIPTION

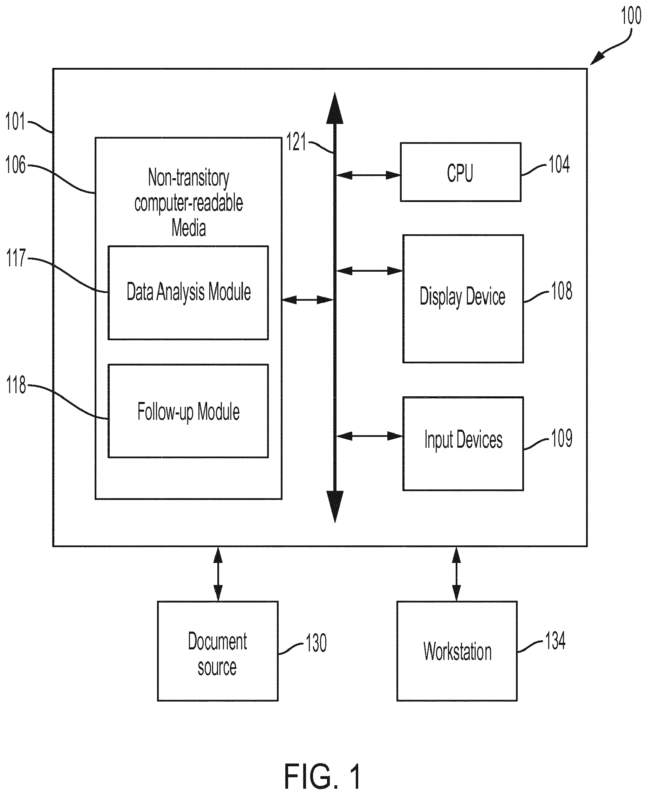

FIG. 1 is a block diagram illustrating an exemplary system 100. The system 100 includes a computer system 101 for implementing the framework as described herein. In some implementations, computer system 101 operates as a standalone device. In other implementations, computer system 101 may be connected (e.g., using a network) to other machines, such as document source 130 and workstation 134. In a networked deployment, computer system 101 may operate in the capacity of a server (e.g., in a server-client user network environment, a client user machine in server-client user network environment, or as a peer machine in a peer-to-peer (or distributed) network environment.

In one implementation, computer system 101 includes a processor device or central processing unit (CPU) 104 coupled to one or more non-transitory computer-readable media 106 (e.g., computer storage or memory device), display device 108 (e.g., monitor) and various input devices 109 (e.g., mouse, touchpad or keyboard) via an input-output interface 121. Computer system 101 may further include support circuits such as a cache, a power supply, clock circuits and a communications bus. Various other peripheral devices, such as additional data storage devices and printing devices, may also be connected to the computer system 101.

The present technology may be implemented in various forms of hardware, software, firmware, special purpose processors, or a combination thereof, either as part of the microinstruction code or as part of an application program or software product, or a combination thereof, which is executed via the operating system. In some implementations, the techniques described herein are implemented as computer-readable program code tangibly embodied in one or more non-transitory computer-readable media 106. In particular, the present techniques may be implemented by a data analysis module 117 and a follow-up module 118. Non-transitory computer-readable media 106 may include random access memory (RAM), read-only memory (ROM), magnetic floppy disk, flash memory, and other types of memories, or a combination thereof. The computer-readable program code is executed by CPU 104 to process data provided by, for example, document source 130. As such, the computer system 101 is a general-purpose computer system that becomes a specific-purpose computer system when executing the computer-readable program code. The computer-readable program code is not intended to be limited to any particular programming language and implementation thereof. It will be appreciated that a variety of programming languages and coding thereof may be used to implement the teachings of the disclosure contained herein.

The same or different computer-readable media 106 may be used for storing image datasets, knowledge base, individual patient data, database of medical records, diagnostic reports or documents for patients, and so forth. Such data may also be stored in an external document source 130. Document source 130 may be implemented using a database management system (DBMS) and residing on a memory, such as a hard disk, RAM, or removable media. Document source 130 may be implemented on one or more additional computer systems (e.g., cloud storage). For example, document source 130 may include a data warehouse system residing on a separate computer system, a picture archiving and communication system (PACS), electronic medical records (EMR) system, radiology information system (RIS), laboratory information system (LIS), or any other now known or later developed hospital, medical institution, medical office, testing facility, pharmacy or other medical patient record storage system.

The workstation 134 may include a computer and appropriate peripherals, such as a keyboard and display device, and can be operated in conjunction with the entire system 100. For example, the workstation 134 may communicate with document source 130 so that the documents or reports retrieved from document source 130 can be presented or displayed at the workstation 134. The workstation 134 may communicate directly with the computer system 101 to display processed data and/or output results. The workstation 134 may include a graphical user interface to receive user input via an input device (e.g., keyboard, mouse, touch screen voice or video recognition interface, etc.) to manipulate visualization and/or processing of the data.

It is to be further understood that, because some of the constituent system components and method steps depicted in the accompanying figures can be implemented in software, the actual connections between the systems components (or the process steps) may differ depending upon the manner in which the present framework is programmed. Given the teachings provided herein, one of ordinary skill in the related art will be able to contemplate these and similar implementations or configurations of the present framework.

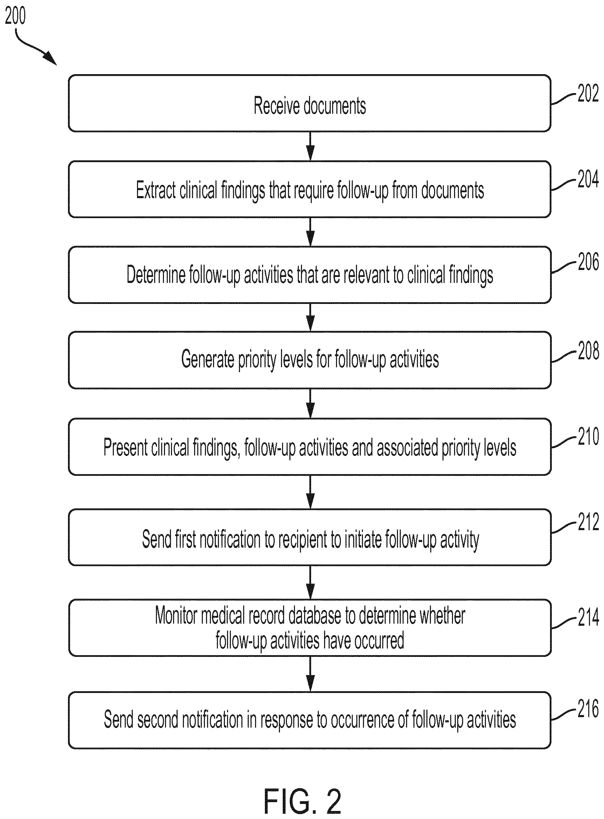

FIG. 2 shows an exemplary method 200 performed by a computer system. It should be understood that the steps of the method 200 may be performed in the order shown or a different order. Additional, different, or fewer steps may also be provided. Further, the method 200 may be implemented with the system 100 of FIG. 1, a different system, or a combination thereof.

At 202, data analysis module 117 receives and reads one or more input documents from document source 130. The input documents may be provided in one or more formats, such as plain or free text, relational database file format, JavaScript Object Notation (JSON), Extensible Markup Language (XML), etc. Free text documents contain unstructured data (e.g., text, graphics, images) that is not organized in a pre-defined manner. The documents may contain diagnostic reports (e.g., radiology reports, pathology reports, colonoscopy reports, laboratory reports), clinical notes (e.g., discharge summaries, progress notes, admission notes, operative notes, primary care notes, etc.), electronic patient data, hospital records, and/or other types of medical data.

At 204, data analysis module 117 extracts, from the one or more input documents, one or more clinical findings that require follow-up. Clinical findings that require follow-up may include, for example, incidental findings, abnormal results from diagnostic tests, and/or a particular medical condition or disease (e.g., chronic diabetes, chronic heart failure) that requires routine clinical follow-up activities for management.

The clinical findings may be extracted from the one or more input documents by using Natural Language Processing (NLP) techniques, Computer-Aided Detection (CAD), discrete structured data capture, or a combination thereof. The extracted clinical findings may be validated by further extracting and analyzing patient factors from electronic records that may enhance clinical understanding or provide relevant clinical context. For example, to validate the presence of a possible incidental finding identified in a radiology report, electronic patient records are analyzed to determine if there are any previous diagnoses related to that finding, existing medical conditions that are related to the finding, etc. Other patient factors (e.g., comorbid conditions) relevant to the classification of a clinical finding may also be extracted from the electronic patient data, as well as discrete structured data that may be implicitly inferred (or captured) rather than explicitly indicated in the patient records.

In some implementations, data analysis module 117 includes a collection processing manager (CPM) for managing the execution of a workflow. CPM may be an XML file that contains three parts: a collection reader to read the input files, analysis engines that performs various Natural Language Processing (NLP) tasks on the input file and a consumer unit that write the results into a file. The analysis engines may include various modules that perform various independent NLP tasks, such as sentence detection, lexical feature generation (e.g., tokenization, part-of-speech or POS tagging, chunking), line sentence detection, lexical variant generation, section segmentation, information extraction, context detection, normalization, etc. In addition, data analysis module 117 may be customized for individual cases. Data analysis module 117 may include a set of regular expression rules and dictionary terms to match against the desired concepts and meta information to be extracted from the input documents. Data analysis module 117 may also apply these NLP tasks in a retrospective analysis of historical files, as well as during real-time monitoring of such files.

At 206, data analysis module 117 determines one or more follow-up activities that are relevant to the one or more extracted clinical findings. Follow-up activities may include, for example, a physical examination, a radiology imaging, bloodwork, medical tests, return visits or any other action to be taken after hospitalization, therapy or diagnosis. Recommendations for follow-up activities may be extracted from the input documents. These recommendations may be provided by, for example, the care provider ordering the examination, a care coordinator or a specialist. If no specific follow-up recommendations are provided in the documents, to the extent that the relevant information may be extracted and/or derived from information that is available for extraction based on the identification of quantitative and qualitative attributes, including the extraction of structured or discrete data fields, data analysis module 117 may generate recommendations in accordance with relevant guidelines for that particular clinical finding. The guidelines may be configurable via a user interface based on user-defined rules. If recommendations of follow-up activities are provided and extracted, to the extent that the relevant information can be extracted from the documents, data analysis module 117 may determine whether the recommendations provided in the patient records are consistent with guidelines.

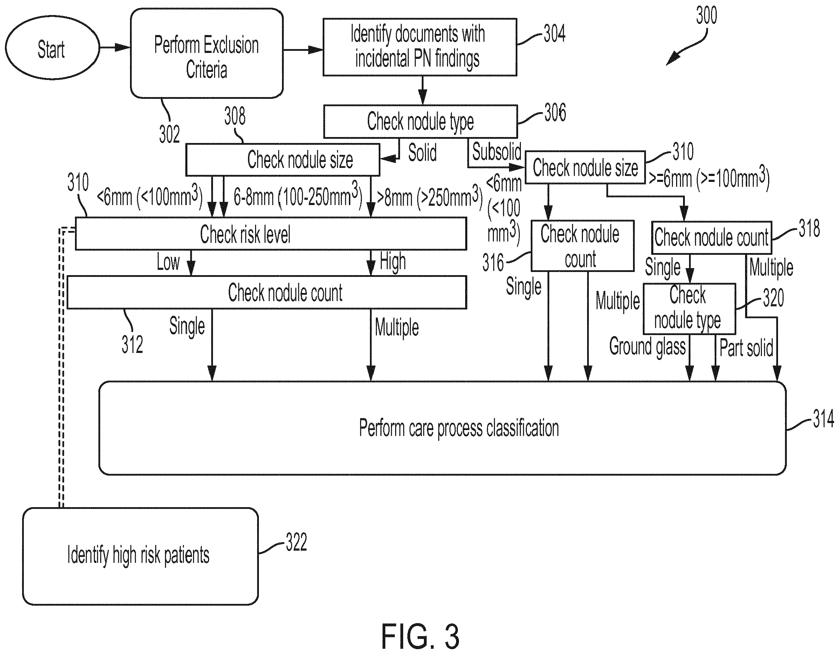

FIG. 3 shows an exemplary decision tree 300 for clinical knowledge extraction. The clinical knowledge may be extracted from, for example, unstructured medical records. Follow-up recommendations may be generated based on established guidelines. In order to generate a follow-up recommendation, additional attributes may be extracted from the documents so that clinical meaning may be applied. Using a clinical case of incidental pulmonary nodules (PN) as an example, the framework extracts the information as identified. Other types of complex decision trees and associated guidelines for a wide variety of clinical conditions may similarly be supported.

At 302, data analysis module 117 performs exclusion criteria on the documents. Exclusion criteria may be determined by the relevant clinical guidelines being applied. Additionally, or alternatively, exclusion criteria may also be based on prioritization logic as will be explained later, whereby documents associated with extremely low priority level (i.e., below a predetermined threshold priority level) patients may be excluded. For example, data analysis module 117 may exclude documents (e.g., medical records) associated with patients that are older than a predetermined age (e.g., 35), in hospice care, has a history of lung cancer, or taking palliative medicine.

At 304, data analysis module 117 identifies documents associated with patients with incidental PN findings. Multiple data sources may be utilized to determine whether a PN finding is incidental, or if it was previously diagnosed with associated follow-up actions that occurred. Such data sources may include, but are not limited to, insurance claims database, information extracted from the EMR related to prior PN diagnosis, latent information obtained from metadata of radiology reports, narrative and structured information extracted from clinical records (e.g., as radiology reports, patient self-reported data).

At 306, based on information contained in the identified documents, data analysis module 117 checks the type of pulmonary nodule. If the nodule is a solid type, data analysis module 117 proceeds to 308 to check the size of the pulmonary nodule. The size may be <6 mm (<100mm³), 6-8mm (100-250mm³) or >8mm (>250mm³).

At 310, data analysis module 117 checks the risk level of the patient (e.g., low or high). At 322, data analysis module 117 identifies high risk patients. Patients may be classified as "high risk level" if any of the following conditions is satisfied: (1) Age >=65; (2) smoker or former smoker; (3) exposure to asbestos, uranium, radon; (4) emphysema or chronic obstructive pulmonary disease (COPD); (5) pulmonary fibrosis; (6) spiculated nodule contour; or (7) nodule located at upper lobe. At 312, data analysis module 117 checks the nodule count (e.g., single or multiple).

If the nodule is a subsolid type, data analysis module 117 proceeds to 310 to check the size of the pulmonary nodule. If the size is <6 mm (<100mm³), data analysis module 117 proceeds to 316 to check the nodule count (e.g., single or multiple). If the size is >=6 mm (>=100mm³), data analysis module 117 proceeds to 318 to check nodule count. If the nodule count is "single", data analysis module 117 proceeds to 320 to check the nodule type (e.g., ground glass or partially solid). If the nodule count is "multiple", data analysis module 117 proceeds to 314 to perform care process classification.

At 314, data analysis module 117 performs care process classification and satisfier checks. For example, data analysis module 117 may check the last radiology imaging (e.g., CT, MR) date, status post lung nodule removal date, status post lung biopsy date, PET/PET-CT, etc. Data analysis module 117 may provide recommendations for follow-up activities based on the care process classification and satisfier checks, with monitoring, to determine if the recommended follow-up activity has been performed (in the case of a retrospective analysis), or if the recommended follow-up activity occurs in the future. For example, if radiology imaging has not been recently performed, a recommendation for performing radiology imaging may be generated.

Returning to FIG. 2, at 208, data analysis module 117 generates one or more priority levels for the one or more follow-up activities. Priority levels may be generated based on user-configurable logic. For example, priority levels may be generated based on likelihood of a follow-up activity not occurring, which can be determined through predictive modeling techniques or any other user-defined criteria. Other factors that may be used to determine the priority level include lack of a primary care physician, clinical context of the finding (e.g., emergency department versus routine physical examination), patient's access to transportation, mental health status, etc. Priority levels provide an additional option for sorting and/or triaging, which is useful particularly when a user is presented with a large number of patients requiring follow-up activities. This capability ensures that patient care can be efficiently coordinated through appropriate management of resources.

At 210, follow-up module 118 presents the one or more extracted clinical findings, follow-up activities and associated priorities. In some implementations, such information is presented in a graphical representation (e.g., table, chart) via, for example, a user interface displayed at workstation 134. The follow-up activities may be sorted based on various criteria, such as type of clinical finding, patient name, priority level, primary care physician (or physician who is otherwise responsible for the patient's care), physician who ordered the examination, and/or a variety of other factors. Different graphical representations may be customized based on the role of the user. For example, a primary care physician may be presented with a graphical representation limited to his/her own patients requiring follow-up, while a care coordinator may be presented with a graphical representation showing all patients requiring follow-up. Follow-up module 118 may further sort recommended follow-up activities based on their due dates. The due dates may be automatically or manually assigned.

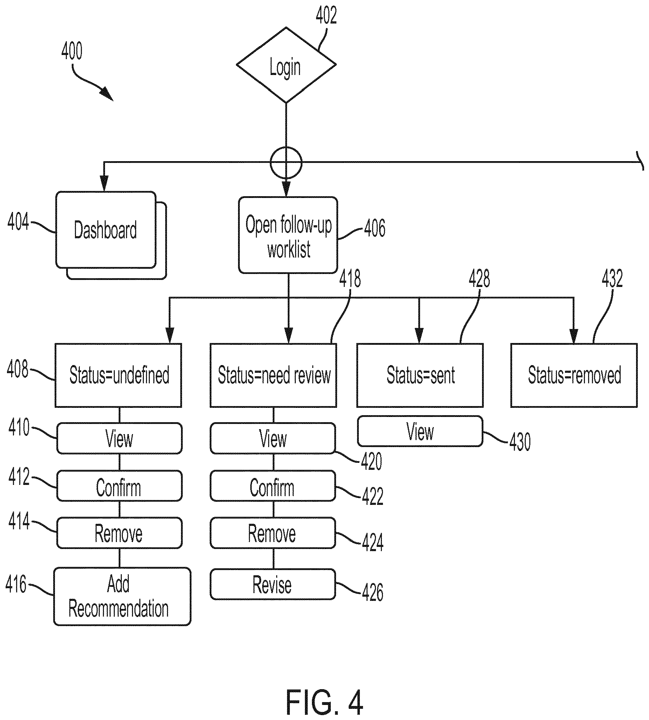

FIG. 4 shows an exemplary workflow 400 for a user who is the rendering care provider. This rendering care provider may be the person who performed the patient examination, created the diagnostic report, etc. The workflow 400 may be facilitated by the user interface generated by the follow-up module 118 and displayed at workstation 134. At 402, the user logs into the user interface. At 404, one or more dashboards are displayed. The dashboard may present an overview of follow-up related information in, for example, a graphical representation (e.g., table, line graph, bar chart). The information may include, but are not limited to, the number of patients who have been identified as needing a follow-up activity or event, the percentage of follow-ups that have occurred, an overview of clinical findings that the follow-up module 118 has identified as requiring follow-up, trends over user-defined periods of time, clinical guidelines adherence rates, etc.

At 406, the user may open a follow-up worklist generated by follow-up module 118 via the user interface. The follow-up worklist identifies the status of each of the follow-up activities associated with clinical findings of patients that the user is able to view based on configurable permissions. At 408, the status of a follow-up activity for a given clinical finding is "undefined". This occurs when the follow-up module 118 is unable to determine a recommendation for a follow-up activity associated with the clinical finding. At 410, the user is able to review the clinical finding and other information associated with the clinical finding extracted by follow-up module 118. At 414, the user can remove the clinical finding if the extraction is incorrect. At 416, the user can manually add the recommendation for a follow-up activity. At 412, the user can confirm the recommendation for the follow-up activity.

At 418, the status of a follow-up activity for a given clinical finding is "need review". At 420, the user is able to review the clinical finding, follow-up activity and other information associated with the clinical finding extracted by follow-up module 118. At 422, the user can confirm the recommendation for a follow-up activity. At 426, the user can revise the recommendation for the follow-up activity.

At 428, the status of a follow-up activity for a given clinical finding is "sent". This means that notification for initiating the follow-up activity is routed to a responsible care provider (i.e., subsequent user) to review the status of the follow-up activity (at 412 or 422). At 430, the initial user is able to view the follow-up activity and other information associated with the follow-up activity. At 424, the user or follow-up module 118 may indicate that the follow-up activity has been completed or otherwise needs to be "removed". Alternatively, follow-up module 118 may automatically detect the completion of the follow-up activity through, for example, data feeds from electronic systems utilized for ordering, scheduling, performing medical examinations or a combination thereof. When the follow-up activity is removed, its status is set to "removed" at 432. At this point, the follow-up activity no longer needs to be tracked, and is available as a historical record.

Returning to FIG. 2, at 212, follow-up module 118 sends a first notification (or reminder) to a recipient to initiate the one or more follow-up activities. The recipient may be the primary care physician (or physician who is otherwise responsible for the patient's care), physician who ordered the examination, care coordinator, specialist, etc. Based on configurable permissions, a user may be able to assign ownership of the follow-up activity and send notifications (or reminders) to the follow-up owner and/or any number of configurable recipients at pre-determined times (e.g., daily, weekly, monthly, before due dates). The follow-up notification may enable the user to initiate automatic generation of an order for the recommended follow-up activity, to the extent a follow-up requires a physician order (e.g., bloodwork, prescription drugs, radiology imaging). The follow-up notification may also include further information, such as recommendations and supporting guidelines. The follow-up notification may be sent via electronic mail (e-mail), text messaging, telephone, social media messaging, or any other communication channel.

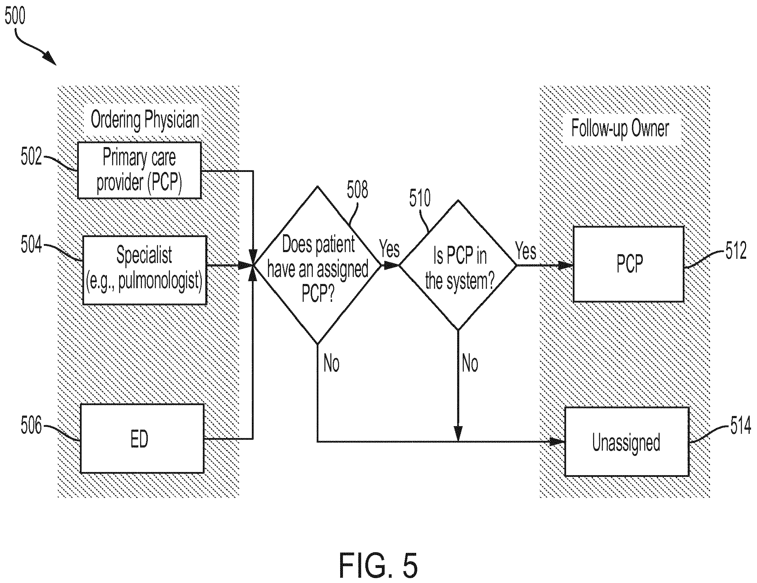

FIG. 5 shows an exemplary method 500 of automatically assigning ownership of a follow-up activity to a responsible care provider. Ownership of each follow-up activity may be assigned to the most relevant clinical care provider (e.g., primary care physician, physician who ordered the examination, specialist). The default state for how the follow-up module 118 automatically routes a follow-up activity and the ability to configure the assignment rules is illustrated. Follow-up module 118 may first determine whether the physician who ordered the examiner is a primary care provider (PCP) 502, a specialist 504, or member of the emergency department (ED) care team 506. At 508, follow-up module 118 determines whether the patient has an assigned PCP. If no, the follow-up activity becomes unassigned at 514. If yes, follow-up module 118 proceeds to 510 to determine if the PCP is a user in this system. If no, the follow-up activity becomes unassigned at 514. If yes, the follow-up activity is assigned to the PCP at 512. Follow-up activities that are unassigned may be manually reviewed in a separate list, so as to determine the appropriate assignment.

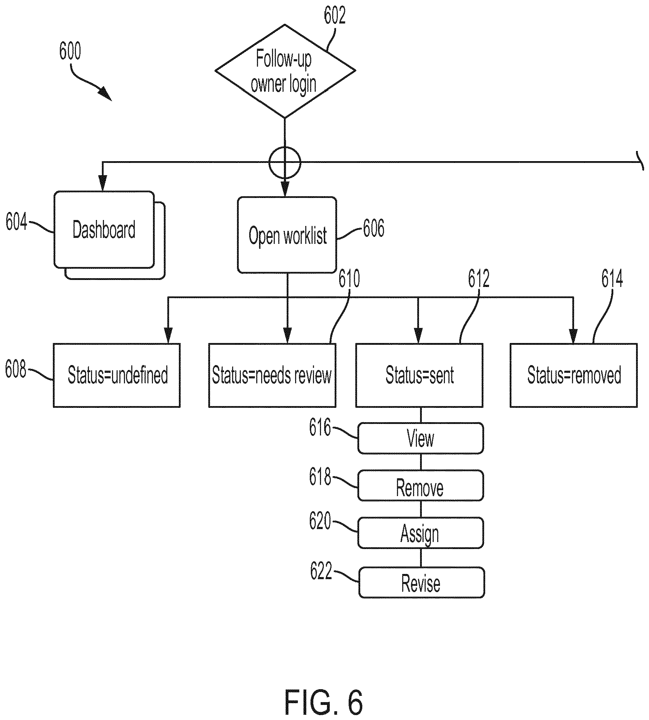

FIG. 6 shows an exemplary workflow 600 for a user who is the owner of a follow-up activity or is otherwise responsible for ensuring that the follow-up occurs as recommended. The follow-up owner may be, for example, the care provider who ordered the examination, the primary care physician, a specialist to whom the patient is the referred, a care coordinator, etc. The workflow 600 may be facilitated by a user interface generated by the follow-up module 118 and displayed at workstation 134.

At 602, the follow-up owner logs into the user interface. At 604, one or more dashboards are displayed to the user. The dashboards may present an overview of information related to the follow-up activities, such as the number of patients who have been identified as needing a follow-up activity, the percentage of follow-up activities that has occurred, an overview of the clinical findings that the follow-up module 118 has identified as requiring follow-up, trends over user-defined periods of time, etc.

At 606, the user is able to open a worklist. The worklist may identify follow-up activities for only patients under the user's care. The user may review any new or existing follow-up activities for the patients displayed in the worklist and the status of the follow-up activities. At 608, the status of the follow-up activity is "undefined" (i.e., follow-up module 118 cannot determine any recommendation for follow-up). At 610, the status of the follow-up activity is "needs review". At 612, the status of the follow-up activity is "sent" (i.e., notification is routed to follow-up owners). At 606, the user may review information associated with the follow-up activity. At 618, the user or follow-up module 118 may indicate that the follow-up activity has been completed or otherwise needs to be removed. At this point the follow-up activity no longer needs to be tracked, and is available as a historical record (with the status of "removed" at 614). At 620, the user may assign the ownership of the follow-up activity to a different care provider. At 622, the user may revise the follow-up activity.

Returning to FIG 2, at 214, follow-up module 118 monitors a medical record database to determine whether the one or more follow-up activities have occurred. The medical record database may be stored in document source 130, non-transitory computer-readable media 106 or another other internal or external data source. The medical record database may be, for example, an electronic medical record (EMR) database, electronic system utilized for ordering, scheduling or performing medical examinations, or a combination thereof. Follow-up module 118 may monitor the medical record database to determine, for example, whether an order (e.g., physician order of examination, bloodwork, imaging, prescription medicine) for the follow-up activity has been placed, and if possible, if the follow-up activity itself has been scheduled, performed, finalized, or otherwise occurred.

At 216, follow-up module 118 sends a second notification in response to occurrence of the one or more follow-up activities. The second notification may be sent via electronic mail (e-mail), text messaging, telephone, social media messaging, or any other communication channel. Follow-up module 118 may further update the status of the follow-up activity to, for example, "removed", so as to remove the follow-up activity from the associated worklists and stop the sending of any further notifications or reminders. Cascading logic may be configured to support specific workflow options. For example, a follow-up activity may be escalated from the initial owner to a secondary owner if the follow-up activity is not removed or closed by the due date. One or more notifications may then be sent to the secondary owner to initiate the follow-up activity.

While the present framework has been described in detail with reference to exemplary embodiments, those skilled in the art will appreciate that various modifications and substitutions can be made thereto without departing from the spirit and scope of the invention as set forth in the appended claims. For example, elements and/or features of different exemplary embodiments may be combined with each other and/or substituted for each other within the scope of this disclosure and appended claims.

## Claims

1. A clinical follow-up system, comprising:
a non-transitory memory device for storing computer readable program code; and
a processor device in communication with the memory device, the processor being operative with the computer readable program code to perform steps including
extracting one or more clinical findings that require follow-up from one or more documents,
determining one or more follow-up activities that are relevant to the one or more clinical findings,
generating one or more priority levels for the one or more follow-up activities,
generating a user interface that presents the one or more clinical findings, the one or more follow-up activities and the one or more priority levels, and
sending a notification to a recipient to initiate at least one of the one or more follow-up activities.

2. The system according to claim 1, wherein the processor is operative with the computer readable program code to extract the one or more clinical findings that require follow-up from the one or more documents using structured data capture, Natural Language Processing (NLP) techniques, or a combination thereof.

3. The system according to claim 1 or 2, wherein the processor is operative with the computer readable program code to further validate the one or more extracted clinical findings by analyzing patient factors in the one or more documents that provides relevant clinical context.

4. A computer-implemented method, comprising:
extracting one or more clinical findings that require follow-up from one or more documents;
determining one or more follow-up activities that are relevant to the one or more clinical findings;
sending a first notification to a recipient to initiate at least one of the one or more follow-up activities;
monitoring a medical record database to determine whether the at least one of the one or more follow-up activities has occurred; and
sending a second notification in response to occurrence of the at least one of the one or more follow-up activities.

5. The method according to claim 4, wherein extracting the one or more clinical findings that require follow-up from the one or more documents comprises extracting the one or more clinical findings that require follow-up from one or more unstructured medical records, and/or
wherein extracting the one or more clinical findings that require follow-up from the one or more documents comprises using structured data capture, Natural Language Processing (NLP) techniques or a combination thereof,
and/or
wherein extracting the one or more clinical findings that require follow-up from the one or more documents comprises extracting an incidental finding, abnormal results from diagnostic tests, a medical condition that requires routine clinical follow-up activities for management, or a combination thereof.

6. The method according to claims 4 or 5, wherein determining the one or more follow-up activities that are relevant to the one or more clinical findings comprises extracting a recommendation for the one or more follow-up activities from the one or more documents,
and/or
wherein determining the one or more follow-up activities that are relevant to the one or more clinical findings comprises generating a recommendation for the one or more follow-up activities in accordance with relevant guidelines for the one or more clinical findings.

7. The method according to any of the preceding claims 4 to 6, further comprises generating one or more priority levels for the one or more follow-up activities, in particular wherein generating one or more priority levels for the one or more follow-up activities comprises generating the one or more priority levels based on likelihood of the one or more follow-up activities not occurring.

8. The method according to any of the preceding claim 4 to 7, further comprises presenting the one or more clinical findings and the one or more follow-up activities in a graphical representation displayed at a user interface, in particular, wherein the method further comprises enabling a user to review, confirm, remove or modify the one or more clinical findings or follow-up activities via the user interface.

9. The method according to claim 8, further comprises enabling a user to assign ownership of the one or more follow-up activities via the user interface.

10. The method according to claims 8 or 9, wherein the graphical representation comprises a worklist that identifies follow-up activities for only patients under a user's care.

11. The method according to claim 10, further comprises sorting the follow-up activities identified by the worklist according to priority levels.

12. The method according to any of the preceding claims 4 to 11, further comprises automatically assigning ownership of the one or more follow-up activities to one or more responsible care providers.

13. The method according to any of the preceding claims 4 to 12, wherein sending the first notification to the recipient to initiate the at least one of the one or more follow-up activities comprises sending the first notification that enables the recipient to initiate automatic generation of an order for the at least one of the one or more follow-up activities.

14. The method according to any of the preceding claim 4 to 13, wherein monitoring the medical record database to determine whether the at least one of the one or more follow-up activities has occurred comprises monitoring the medical record database to determine whether an order for the follow-up activity has been placed.

15. One or more non-transitory computer readable media embodying a program of instructions executable by machine to perform steps comprising:
extracting one or more clinical findings that require follow-up from one or more documents;
determining one or more follow-up activities that are relevant to the one or more clinical findings;
sending a first notification to a recipient to initiate at least one of the one or more follow-up activities;
monitoring a medical record database to determine whether the at least one of the one or more follow-up activities has occurred; and
sending a second notification in response to occurrence of the at least one of the one or more follow-up activities.
